# EUROPEAN PATENT APPLICATION

(11) **EP 4 692 040 A1**
(43) Date of publication of application: **11.02.2026**
(21) Application number: 24868502.6
(22) Date of filing: 26.08.2024
(51) Int. Cl.: C07C 51/43, C07C 57/04, C07C 51/25

(54) **METHOD FOR PREPARING ACRYLIC ACID**

(30) Priority: 22.09.2023 KR 20230126983; 21.08.2024 KR 20240112157
(71) Applicant: LG CHEM, LTD., Seoul 07336 (KR)
(72) Inventor: YOO, Sung Jin, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2024/012704
(87) International publication number: WO 2025/063523

(57) **Abstract**

Provided is a method for preparing an acrylic acid including: bringing a mixed gas including an acrylic acid into contact with water in an absorption tower to obtain an acrylic acid solution including the acrylic acid, in which crystallization the acrylic acid is performed by including a multistage crystallization process in which two or more crystallization units including a crystallizer and a solid-liquid separator are connected in series.

## Description

### [Technical Field]

### CROSS-REFERENCE TO RELATED APPLICATIONS

The present application claims the benefit of priorities to Korean Patent Application No. 10-2023-0126983, filed on September 22, 2023 and Korean Patent Application No. 10-2024-0112157, filed on August 21, 2024, the entire contents of which are incorporated herein as a part of the specification.

### Technical Field

The present invention relates to a method for preparing an acrylic acid, and more particularly, to a method for preparing a high-purity acrylic acid according to a continuous crystallization process.

### [Background Art]

An acrylic acid is generally prepared by a method of subjecting a compound such as propane, propylene, and acrolein to a gaseous oxidation reaction in the presence of a catalyst. For example, propane, propylene, and the like are converted through acrolein into an acrylic acid by a gaseous oxidation reaction in the presence of an appropriate catalyst in a reactor, and a mixed gas including an acrylic acid, unreacted propane or propylene, acrolein, inert gas, carbon dioxide, water vapor, and various organic by-products by the reaction (such as acetic acid, low-boiling point by-products, and high-boiling point by-products) is obtained in an after-stage of the reactor.

The acrylic acid-containing mixed gas is recovered as an acrylic acid aqueous solution by an absorption process of contacting with an absorption solvent such as water in an absorption tower. Herein, when absorption efficiency in the absorption process is not good, a large amount of the acrylic acid may be lost in an upper portion of the absorption tower in which the absorption process is performed.

Thereafter, it is common to involve a process such as crystallization, extraction, distillation, and purification as a subsequent process for obtaining the acrylic acid included in the acrylic acid aqueous solution.

A crystallization process may be involved in order to obtain the acrylic acid included in the acrylic acid solution, but since an acetic acid as an organic by-product has a melting point similar to the acrylic acid, when a large amount of the acetic acid is introduced to the crystallization process, an acrylic acid having targeted purity may not be obtained by only one-stage crystallization process.

Therefore, there is an urgent need to introduce technology which allows a high-purity acrylic acid to be obtained from the acrylic acid solution, while reducing the amount of acrylic acid lost in the absorption process.

### [Disclosure]

### [Technical Problem]

In order to solve the problems mentioned in the Background Art, an object of the present invention is to provide a method for preparing an acrylic acid which allows an acrylic acid included in an acrylic acid solution to be obtained with high purity, while reducing the amount of the acrylic acid lost in the absorption process.

### [Technical Solution]

In one general aspect, a method for preparing an acrylic acid includes: bringing a mixed gas including an acrylic acid into contact with water in an absorption tower to obtain an acrylic acid solution including the acrylic acid; performing crystallization of the acrylic acid by a process including a multistage crystallization process in which two or more crystallization units including a crystallizer and a solid-liquid separator are connected in series, introducing the acrylic acid solution to the crystallizer of a first stage crystallization unit of the two or more crystallization units; crystallizing the acrylic acid, which is included in the acrylic acid solution introduced to the crystallizer in each stage, in the crystallizer in each stage to obtain a suspension including an acrylic acid crystal, and supplying the suspension to a solid-liquid separator in each stage; solid-liquid separating the suspension in the solid-liquid separator in each stage to obtain a concentrated acrylic acid solution and a mother liquor; diverging the mother liquor into a first mother liquor stream and a second mother liquor stream, circulating the first mother liquor stream to the crystallizer in a prior-stage to a stage to which the solid-liquid separator from which the mother liquor is separated belongs or to the absorption tower, and circulating the second mother liquor stream to the crystallizer in the stage to which the solid-liquid separator from which the mother liquor is separated belongs; introducing the concentrated acrylic acid solution to the crystallizer in an after-stage of a stage to which the solid-liquid separator from which the concentrated acrylic acid solution is separated belongs; and obtaining an acrylic acid crystal from the solid-liquid separator of the crystallization unit in the last stage of the two or more crystallization units.

### [Advantageous Effects]

According to the method for preparing an acrylic acid of the present invention, a purified acrylic acid may be obtained with high purity, by crystallizing an acrylic acid included in an acrylic acid solution through a multistage crystallization process performed by crystallization units which are connected in multiple stages.

In particular, when the two or more crystallization units are in a total of two stages, the amount and the flow rate of the acrylic acid included in the first mother liquor stream diverging from a first stage solid-liquid separator are adjusted by controlling a crystallization temperature in a crystallizer of a first stage crystallization unit, so that absorption efficiency in the absorption tower may be maintained similar to previous absorption efficiency. Furthermore, it may be preferred in terms of using a refrigerant in the crystallizer, and the purified acrylic acid may be obtained with high purity by lowering the content of impurities included in a finally obtained purified acrylic acid.

### [Description of Drawings]

FIG. 1 is a process diagram showing the method for preparing an acrylic acid according to an exemplary embodiment of the present invention.
FIGS. 2 to 5 are process diagrams showing methods for preparing an acrylic acid according to the comparative examples.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present invention, the term "stream" may refer to a fluid flow in the process, or may refer to the fluid itself flowing in a moving line (pipe). Specifically, the "stream" may refer to both a fluid itself flowing in a pipe connecting each device and a fluid flow. In addition, the fluid may refer to inclusion of any one or more of gas, liquid, and solid.

The term "upper portion" used herein refers to, unless otherwise stated, a point at 0% to 10% height from the top to the bottom of an apparatus, and specifically, may refer to a top (tower top). In addition, the term "lower" refers to a point at 90% to 100% height from the top to the bottom of an apparatus, and specifically, may refer to a bottom (tower bottom).

In addition, "pressure" mentioned herein refers to gauge pressure measured under atmospheric pressure conditions.

Meanwhile, unless otherwise particularly stated herein, operation pressure of a column refers to pressure in an upper portion of the column, and operation temperature of a column refers to a temperature in a lower portion of the column.

In the method for preparing an acrylic acid according to an exemplary embodiment of the present invention, a mixed gas including an acrylic acid is brought into contact with water in an absorption tower to obtain an acrylic acid solution including the acrylic acid; performing crystallization of the acrylic acid by a process including a multistage crystallization process in which two or more crystallization units including a crystallizer and a solid-liquid separator are connected in series; the acrylic acid solution is introduced to the crystallizer of a first stage crystallization unit of the two or more crystallization units; the acrylic acid, which is included in the acrylic acid solution introduced to the crystallizer in each stage in the crystallizer, in each stage is crystallized to obtain a suspension including an acrylic acid crystal, and the suspension is supplied to a solid-liquid separator in each stage; the suspension is solid-liquid separated in the solid-liquid separator in each stage to obtain a concentrated acrylic acid solution and a mother liquor; the mother liquor diverges into a first mother liquor stream and a second mother liquor stream, the first mother liquor stream is circulated to the crystallizer in a prior-stage to a stage to which the solid-liquid separator from which the mother liquor is separated belongs or to the absorption tower, and the second mother liquor stream is circulated to the crystallizer in the stage to which the solid-liquid separator from which the mother liquor is separated belongs; the concentrated acrylic acid solution is introduced to the crystallizer in an after-stage of a stage to which the solid-liquid separator from which the concentrated acrylic acid solution is separated belongs; and a purified acrylic acid may be obtained from the solid-liquid separator of the crystallization unit in the last stage of the two or more crystallization units.

In the method for preparing an acrylic acid according to an exemplary embodiment of the present invention, a mixed gas including an acrylic acid may be brought into contact with water in an absorption tower to obtain an acrylic acid solution including the acrylic acid.

First, the mixed gas including an acrylic acid may be prepared as follows.

Reactants including a gas containing oxygen and a raw material compound may be supplied to a reactor provided with a catalyst, and a gaseous oxidation reaction may be performed in the presence of a catalyst in the reactor to obtain the mixed gas including an acrylic acid. Herein, the acrylic acid may be a crude acrylic acid, and the gas containing oxygen may be air. The crude acrylic acid may be an acrylic acid before crystallization.

The raw material compound may be one or more compounds selected from the group consisting of propane, propylene, butane, i-butylene, t-butylene, and acrolein, and specifically, the raw material compound may include propylene.

Therefore, the mixed gas including an acrylic acid of the present invention may be a reaction product by the gaseous oxidation reaction of the reactants including air and the raw material compound in the reactor. Specifically, the mixed gas may include an acrylic acid, an unreacted raw material compound, acrolein, inert gas, carbon monoxide, carbon dioxide, various organic by-products (acetic acid, low-boiling point by-products, high-boiling point by-product, etc.), and the like. Herein, "low-boiling point by-products" (light ends) or "high-boiling point by-products" (heavies) are a kind of by-products which may be produced in the preparation process of the target acrylic acid, and may be a compound having a higher or lower molecular weight than the acrylic acid. Thereafter, a mixed gas including the acrylic acid may be supplied to an absorption tower.

Specifically, the mixed gas including the acrylic acid may be brought into contact with water in the absorption tower to obtain an acrylic acid solution. Herein, considering efficiency of the absorption process, the mixed gas may be supplied to the lower portion of the absorption tower, and an absorption solvent, specifically, water may be supplied to the upper portion of the absorption tower.

Herein, the kind of absorption tower may be determined considering contact efficiency of the mixed gas and the absorption solvent and the like, and for example, may be a packed column type absorption tower or a multistage tray type absorption tower. To the inside of the packed column type absorption tower, a filler such as a Rashing ring, a Pall ring, a saddle, a gauze, and a structured packing may be applied.

Meanwhile, the absorption tower may be operated at an internal temperature of 50 to 120°C or 50 to 100°C under an internal pressure of 1 to 1.5 bar or 1 to 1.3 bar, considering a moisture content depending on a saturated vapor pressure and condensation conditions of the acrylic acid, and the like.

As such, a crude acrylic acid aqueous solution including a crude acrylic acid may be obtained as an acrylic acid solution, by an absorption process performed in the absorption tower.

Meanwhile, according to the method for preparing an acrylic acid of the present invention, crystallization of the acrylic acid may be performed by a process including a multistage crystallization process in which two or more crystallization units including a crystallizer and a solid-liquid separator are connected in series.

Specifically, in the multistage crystallization process, the two or more crystallization units are connected in multiple stages, so that the crystallization process performed in the crystallization unit in each stage may be continuously performed. The crystallization unit in each stage may include the crystallizer and the solid-liquid separator, respectively.

The crystallizer may crystallize a specific material into a solid using a difference in solubility of a material depending on a temperature in a liquid mixture, and the solid-liquid separator may separate a solid-liquid mixture supplied to the solid-liquid separator into a solid phase and a liquid phase.

Meanwhile, according to the method for preparing an acrylic acid of the present invention, the acrylic acid solution may be introduced to the crystallizer of a first stage crystallization unit among the two or more crystallization units.

Specifically, the crude acrylic acid aqueous solution obtained in the absorption tower may be introduced to the crystallizer of the first stage crystallization unit among the two or more crystallization units, as the acrylic acid solution. As described above, the crude acrylic acid aqueous solution may include the crude acrylic acid which is an acrylic acid before crystallization.

Meanwhile, the first stage crystallization unit may be a crystallization unit placed in the very first stage among the two or more crystallization units, and may be in a stage to which a crystallizer to which the crude acrylic acid aqueous solution is introduced belongs.

According to the present invention, the amount of the acrylic acid included in the acrylic acid solution introduced to the crystallizer of the first stage crystallization unit may be 80 to 95 wt%, specifically 82 wt% to 90 wt%. A crystallization temperature of the acrylic acid in the crystallizer in each stage may vary depending on the amount of the acrylic acid in the acrylic acid solution introduced to the crystallizer of each crystallization unit, and the amount of the acrylic acid crystal produced may vary depending on the temperature.

More specifically, when the amount of the acrylic acid in the acrylic acid solution introduced to the crystallizer of the first stage crystallization unit is less than 80 wt%, the content of the acrylic acid in the acrylic acid solution is low, and thus, when the acrylic acid solution is supplied to the crystallizer, the amount of the acrylic acid crystal obtained by the crystallization may also be small, and a process performed for satisfying the purity and the yield of the acrylic acid to a level to be desired in the present invention may become complicated. Meanwhile, when the amount of the acrylic acid in the acrylic acid solution introduced to the crystallizer of the first stage crystallization unit is higher than 95 wt%, an additional purification process may be required to obtain the acrylic acid solution. Therefore, it may not be preferred from the point of view of economic feasibility such as process equipment and device costs for performing the purification process, and an amount of energy used may also be excessively increased.

According to the method for preparing an acrylic acid of the present invention, the acrylic acid included in the acrylic acid solution introduced to the crystallizer in each stage is crystallized in the crystallizer in each stage to obtain a suspension including an acrylic acid crystal, and the suspension may be supplied to the solid-liquid separator in each stage, specifically, the solid-liquid separator in the stage to which the crystallizer from which the suspension is obtained belongs.

More specifically, the crude acrylic acid aqueous solution obtained as described above may be introduced to the crystallizer of the first stage crystallization unit as the acrylic acid solution. Meanwhile, the concentrated acrylic acid solution separated from the solid-liquid separator in the prior-stage may be introduced as the acrylic acid solution to the crystallizer of the crystallization unit following the first stage crystallization unit, that is, the crystallizer of the crystallization unit from the second stage. Herein, the crystallization unit following the first stage crystallization unit may refer to all crystallization units following the first stage crystallization unit among the two or more crystallization units. For example, when the crystallization units are in a total of four stages, the crystallization units following the first stage crystallization unit may be a second stage crystallization unit, a third stage crystallization unit, and a fourth stage crystallization unit.

Therefore, the crystallization unit following the first stage crystallization unit may have a higher amount of the acrylic acid included in the concentrated acrylic acid solution introduced to the crystallizer of each crystallization unit as the crystallization unit is in a later stage.

Meanwhile, the acrylic acid included in the acrylic acid solution is crystallized by a crystallization process performed in the crystallizer in each stage, thereby obtaining a suspension including an acrylic acid crystal. Thereafter, the suspension obtained in the crystallizer in each stage may be supplied to the solid-liquid separator in the stage to which the crystallizer from which the suspension is obtained belongs.

According to an exemplary embodiment of the present invention, a ratio of the mass of the acrylic acid crystal included in the suspension to the mass of the suspension discharged from the crystallizer in each stage may be 10 to 40 wt%, specifically 20 to 35 wt%. The ratio of the mass of the acrylic acid crystal included in the suspension to the total mass of the suspension discharged from the crystallizer in each stage may have the same meaning as a ratio of a mass flow rate of the crystallized acrylic acid included in the suspension to the mass flow rate of the suspension supplied to the solid-liquid separator in each stage.

More specifically, when the ratio of the mass of the acrylic acid crystal included in the suspension to the mass of the suspension discharged from the crystallizer in each stage, that is, the acrylic acid crystal content in the suspension is less than 10 wt%, it is in a state in which a relatively small amount of acrylic acid is crystallized in the crystallizer in each stage, and thus, in order to satisfy the acrylic acid production to a desired level, the total number of the multistage crystallization processes and the throughput may be increased. When the acrylic acid crystal content in the suspension is more than 40 wt%, the concentration of the acrylic acid crystal which is a solid in the suspension is increased, so that it may be difficult to stir the suspension inside the crystallizer in each stage. Accordingly, since it may also be difficult to move the suspension to the solid-liquid separator, it may be difficult to perform the crystallization process.

According to the method for preparing an acrylic acid of the present invention, the suspension may be solid-liquid separated in the solid-liquid separator in each stage to obtain a concentrated acrylic acid solution and a mother liquor.

Specifically, in the solid-liquid separator in each stage, the suspension obtained from the crystallizer in each stage may be solid-liquid separated, respectively, to obtain a solid acrylic acid crystal and a liquid mother liquor. The mother liquor may be a remainder after separating the acrylic acid crystal from the suspension, and may include an uncrystallized acrylic acid, water, and an acetic acid.

According to an exemplary embodiment of the present invention, obtaining the concentrated acrylic acid solution in the solid-liquid separator at each stage may be performed by including solid-liquid separating the suspension in the solid-liquid separator in each stage to obtain an acrylic acid crystal, and melting the acrylic acid crystal to obtain the concentrated acrylic acid solution.

Specifically, in the solid-liquid separator in each stage, the suspension obtained from the crystallizer in each stage may be solid-liquid separated, respectively, to obtain a solid acrylic acid crystal and a liquid mother liquor. The mother liquor may be a remainder after separating the acrylic acid crystal from the suspension, and may include an uncrystallized acrylic acid, water, and an acetic acid.

Furthermore, the concentrated acrylic acid may be obtained by solid-liquid separating the suspension in the solid-liquid separator in each stage to obtain an acrylic acid crystal and melting the acrylic acid crystal. That is, the acrylic acid crystal may be melted by heat to obtain the concentrated acrylic acid solution. For example, the molten acrylic acid crystal may be introduced to the crystallizer in the after-stage crystallization unit as the concentrated acrylic acid solution.

The melting may be a physical process of phase transition from solid to liquid, and though the acrylic acid crystal is melted, the number of moles of the acrylic acid included in the acrylic acid crystal may not be changed. Herein, in order to melt the acrylic acid crystal, a separate heat supply device, for example, a heat exchanger may be further provided. The concentrated acrylic acid solution may include the acrylic acid, and additionally, may include impurities (such as water and acetic acid) included in the acrylic acid crystal and a small amount of a mother liquor which remains in the acrylic acid crystal without being separated from the solid-liquid separator in each stage.

In order to introduce the acrylic acid crystal separated from the solid-liquid separator in each stage to the crystallizer of the crystallization unit in the after-stage of the stage to which the solid-liquid separator from which the acrylic acid crystal is separated belongs or obtain the acrylic acid crystal, melting may be preferred. That is, the melting of the acrylic acid crystal may be performed for moving of the acrylic acid crystal in the multistage crystallization process, since the acrylic acid crystal is a solid phase.

According to the method for preparing an acrylic acid according to an exemplary embodiment of the present invention, the mother liquor may diverge into a first mother liquor stream and a second mother liquor stream, the first mother liquor stream may be circulated to the crystallizer in a prior-stage to a stage to which the solid-liquid separator from which the mother liquor is separated belongs or the absorption tower, and the second mother liquor stream may be circulated to the crystallizer in the stage to which the solid-liquid separator from which the mother liquor is separated belongs.

Specifically, the mother liquor is a mother liquor separated from the solid-liquid separator in each stage, and may diverge into the first mother liquor stream and the second mother liquor stream.

Thereafter, the first mother liquor stream may be circulated to the absorption tower or the crystallizer in the prior-stage to the stage to which the solid-liquid separator from which the first mother liquor stream originates belongs. Specifically, the first mother liquor stream diverging from the mother liquor obtained from the solid-liquid separator of the first stage crystallization unit may be circulated to the absorption tower. Meanwhile, the first mother liquor stream diverging from the mother liquor obtained from the solid-liquid separator of the crystallization unit following the first stage crystallization unit may be circulated to the crystallizer in the prior-stage to the stage to which the solid-liquid separator from which the mother liquor is obtained belongs.

Meanwhile, the second mother liquor stream may be circulated to the crystallizer of the stage to which the solid-liquid separator from which the second mother liquor stream originates belongs.

According to an exemplary embodiment of the present invention, the mother liquor obtained from the solid-liquid separator of the first stage crystallization unit may diverge into the first mother liquor stream and the second mother liquor stream, the first mother liquor stream diverging from the first stage solid-liquid separator may be circulated to the absorption tower, and the second mother liquor stream diverging from the first stage solid-liquid separator may be circulated to the crystallizer of the first stage crystallization unit.

Specifically, the first stage solid-liquid separator may be a solid-liquid separator included in the first stage crystallization unit. The first mother liquor stream diverging from the first stage solid-liquid separator may be circulated to the absorption tower as the first mother liquor stream, and the second mother liquor stream diverging from the first stage solid-liquid separator may be circulated to the crystallizer of the first stage crystallization unit as the second mother liquor stream.

Meanwhile, absorption efficiency in the absorption tower may be adjusted, depending on the flow rate of the first mother liquor stream diverging from the first stage solid-liquid separator, the amount of the acrylic acid included in the first mother liquor stream diverging from the first stage solid-liquid separator, and the position at which the first mother liquor stream diverging from the first stage solid-liquid separator is circulated to the absorption tower.

Meanwhile, the amount of the acrylic acid included in the first mother liquor stream diverging from the first stage solid-liquid separator may be 65 to 85 wt%, specifically 70 to 80 wt%.

Specifically, in the case in which the amount of the acrylic acid included in the first mother liquor stream diverging from the first stage solid-liquid separator is 85 wt% or less, when the first mother liquor stream is introduced to the absorption tower, it may serve as an absorption solvent for the acrylic acid. In addition, as the flow rate of the first mother liquor stream diverging from the first stage solid-liquid separator is higher and the amount of the acrylic acid included in the first mother liquor stream introduced to the absorption tower is lower, the absorption efficiency in the absorption tower may be improved.

According to the present invention, the position at which the first mother liquor stream diverging from the first stage solid-liquid separator is circulated to the absorption tower may be at 10% to 60% height, specifically 20% to 50% height from the top to the bottom of the absorption tower, based on the top of the absorption tower 100 being 0%.

When the position at which the first mother liquor stream diverging from the first stage solid-liquid separator is circulated to the absorption tower is less than 10%, the first mother liquor stream is circulated to a higher position, and thus, the first mother liquor stream having a higher content of acrylic acid is circulated to the position of the absorption tower at which the absorption solution having a lower content of acrylic acid is present, thereby decreasing the absorption efficiency of the absorption tower. Meanwhile, when the position at which the first mother liquor stream diverging from the first stage solid-liquid separator is circulated to the absorption tower is more than 60%, the first mother liquor stream is circulated to a lower position, and thus, an area in the absorption tower in which the first mother liquor stream may serve as the absorption solvent to absorb the acrylic acid included in the mixed gas is decreased, so that it may be difficult to perform a function as the absorption solvent.

According to an exemplary embodiment of the present invention, the mass flow rate of the first mother liquor stream diverging from the first stage solid-liquid separator may be 5 to 50%, specifically 5 to 30%, of the mass flow rate of the mixed gas including the acrylic acid.

Specifically, when the mass flow rate of the first mother liquor stream diverging from the first stage solid-liquid separator is less than 5% of the mass flow rate of the mixed gas, the flow rate of the first mother liquor stream introduced to the absorption tower is lower, and thus, it may be difficult to absorb the acrylic acid included in the mixed gas in the absorption tower. Meanwhile, when the mass flow rate of the first mother liquor stream diverging from the first stage solid-liquid separator is more than 50% of the mass flow rate of the mixed gas, a device size, for example, the diameter of the absorption tower should be larger, and thus, it may not be preferred in the economic point of view. In addition, the operation capacity of the absorption tower may exceed a design capacity, and in this case, the operation of the absorption tower may become difficult.

According to the method for preparing an acrylic acid according to an exemplary embodiment of the present invention, the concentrated acrylic acid solution may be introduced to the crystallizer in the after-stage of the stage to which the solid-liquid separator from which the concentrated acrylic acid solution is separated belongs.

Specifically, the concentrated acrylic acid solution is obtained by melting the acrylic acid crystal separated from the solid-liquid separator in each stage. The concentrated acrylic acid solution may be introduced to the crystallizer in an after-stage of a stage to which the solid-liquid separator from which the concentrated acrylic acid solution is separated belongs. Therefore, as described above, the concentrated acrylic acid solution may be introduced to the crystallizer of the crystallization unit following the first stage crystallization unit, as the acrylic acid solution.

For example, when the crystallization units are in a total of three stages, the concentrated acrylic acid solution separated from the solid-liquid separator of the second stage crystallization unit may be introduced to the crystallizer of a third stage crystallization unit.

According to the method for preparing an acrylic acid according to an exemplary embodiment of the present invention, a purified acrylic acid may be obtained from the solid-liquid separator of the crystallization unit in the last stage among the two or more crystallization units.

Specifically, the concentrated acrylic acid solution obtained by melting the acrylic acid crystal separated from the solid-liquid separator of the last stage crystallization unit may be obtained as an acrylic acid which is a product of the present invention, that is, a purified acrylic acid. The last stage crystallization unit may refer to a crystallization unit placed in the last stage among the two or more crystallization units connected in multiple stages.

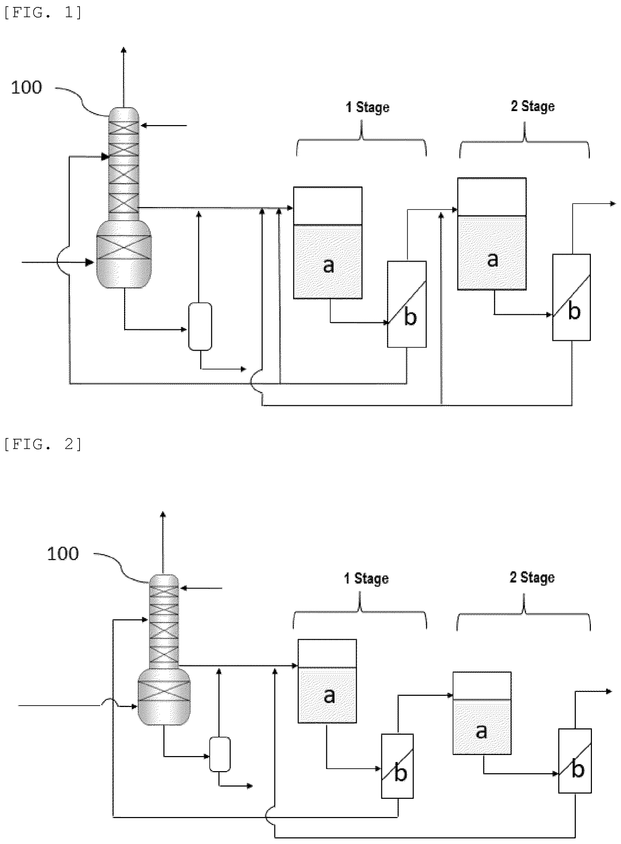

Hereinafter, the process which may be included in the exemplary embodiment of the present invention will be described, with reference to FIG. 1.

According to an exemplary embodiment of the present invention, the two or more crystallization units may be in a total of two stages,
the crystallization temperature in the crystallizer (a) of the first stage crystallization unit may be -9 to 5°C, and the crystallization temperature in the crystallizer (a) of the second stage crystallization unit may be 0 to 12°C. Specifically, the crystallization temperature in the crystallizer (a) of the first stage crystallization unit may be -8 to 0°C, and the crystallization temperature in the crystallizer (a) of the second stage crystallization unit may be 5 to 10°C. Herein, the crystallization temperature may refer to an operation temperature of the crystallizer for crystallizing the acrylic acid solution introduced to the crystallizer in each stage into an acrylic acid crystal.

Specifically, when the two or more crystallization units are in a total of two stages, the first stage crystallization unit and the second stage crystallization unit may be included. The first stage crystallization unit is a crystallization unit including the crystallizer to which the acrylic acid solution obtained in the absorption tower is introduced, and the second stage crystallization unit is a crystallization unit following the first stage crystallization unit.

More specifically, when the crystallization temperature in the crystallizer (a) of the first stage crystallization unit is within the range, the crystallization temperature in the crystallizer of the first stage crystallization unit is relatively high and the amount of the acrylic acid crystallized in the crystallizer (a) of the first stage crystallization unit is reduced, and thus, for the production amount of the acrylic acid targeted in the present invention, the flow rates of the acrylic acid solution introduced to the crystallizer (a) of the first stage crystallization unit from the absorption tower 100 and the mother liquor separated from the solid-liquid separator (b) of the first stage crystallization unit may be increased. Thus, the flow rate of the first mother liquor stream diverging from the first stage solid-liquid separator (b) may also be increased, and the absorption efficiency in the absorption tower 100 may be increased as the flow rate of the first mother liquor stream circulated to the absorption tower is increased. However, as the amount of the acrylic acid crystallized in the crystallizer (a) of the first stage crystallization unit is decreased, the composition of the acrylic acid in the mother liquor separated from the solid-liquid separator (b) of the first stage crystallization unit may be increased, and the absorption efficiency in the absorption tower 100 may be decreased as the composition of the acrylic acid in the first mother liquor stream diverging from the first stage solid-liquid separator (b) is increased.

Therefore, when the crystallization temperature in the crystallizer (a) of the first stage crystallization unit is within the range, decreased absorption efficiency due to the increased composition of the acrylic acid in the first mother liquor stream diverging from the first stage solid-liquid separator (b) and increased absorption efficiency by the increased flow rate of the first mother liquor stream may function together. In this case, loss of the acrylic acid in the absorption tower 100 may be maintained similar to the previous one, and since the temperature of the crystallizer (a) of the first stage crystallization unit is relatively high, it may be favorable in terms of use of a refrigerant (utility) for the crystallization process in the first stage crystallization unit. Furthermore, since the amount of impurities, for example, acetic acid and water, which are concentrated with the acrylic acid in the crystallizer (a) of the first stage crystallization unit is decreased, it may be preferred in terms of purity of the finally obtained purified acrylic acid.

Meanwhile, when the crystallization temperature range in the crystallizer (a) of the first stage crystallization unit is exceeded, the content of the acrylic acid in the first mother liquor stream diverging from the first stage solid-liquid separator (b) is excessively increased, and thus, even when the flow rate of the first mother liquor stream diverging from the first stage solid-liquid separator (b) is high, loss of the acrylic acid in the absorption tower 100 may be increased.

Meanwhile, when the crystallization temperature in the crystallizer of the second stage crystallization unit is within the range, the first and second mother liquor streams diverging from the solid-liquid separator (b) of the second stage crystallization unit are supplied to the crystallizer (a) of the first and second stage crystallization units, so that the acrylic acid crystal content in the suspension discharged from the crystallizer of the first and second stage crystallization units may be maintained at 10 to 40 wt%. In addition, in terms of the use of a refrigerant (utility) for the crystallization process and the purity of acrylic acid, it is preferred to control the crystallization temperature in the crystallizer of the second stage crystallization unit to the above range.

According to an exemplary embodiment of the present invention, a mass flow rate ratio between the first mother liquor stream and the second mother liquor stream diverging from the solid-liquid separator (b) of the first stage crystallization unit may be 1:9 to 1:1, and the mass flow rate ratio between the first mother liquor stream and the second mother liquor stream diverging from the solid-liquid separator (b) of the second stage crystallization unit may be 1:1 to 9:1. In this case, the acrylic acid crystal content in the suspension discharged from the crystallizer (a) in each stage may be controlled to 10 to 40 wt%. Thus, in performing the multistage crystallization process, a stream flow in the crystallization unit or between the crystallization units may be continuously performed, thereby finally obtaining the acrylic acid. Herein, the stream may include, for example, a suspension.

The ranges of the mass flow rate between the first mother liquor stream and the second mother liquor stream diverging from the solid-liquid separator (b) of the first stage crystallization unit and the mass flow rate between the first mother liquor stream and the second mother liquor stream diverging from the solid-liquid separator (b) of the second stage crystallization unit may be determined depending on the crystallization temperature in the crystallizer (a) in each stage and the concentration of the acrylic acid crystal included in the suspension discharged from the crystallizer (a) in each stage.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Examples

### Example 1

According to the process diagram shown in FIG. 1, a preparation process of an acrylic acid was simulated, using an Aspen Plus simulator available from Aspen Technology, Inc. As a crystallizer model, a model on which a distribution coefficient value of water and acetic acid from the solid-liquid equilibrium (SLE) data of acrylic acid and water, acrylic acid and acetic acid, and water and acetic acid and the crystallization experiment results of acrylic acid, water, and acetic acid was reflected was used.

Specifically, a mixed gas including acrylic acid (16.6 wt%), acetic acid (0.46 wt%), and water (6.96 wt%) was brought into contact with water as an absorption solvent in an absorption tower 100 to obtain an acrylic acid solution. The acrylic acid solution was introduced to a multistage crystallization process in which a total of two stages of crystallization units were connected as an absorption tower side discharge stream.

Meanwhile, the absorption tower upper discharge stream including components which were not absorbed in water was discharged outside the system, and the absorption tower lower discharge stream including high-boiling point by-products was supplied to a high-boiling point by-product removal tower.

The absorption tower lower discharge stream was distilled in the high-boiling point by-product removal tower to discharge the high-boiling point by-products outside the system, and the acrylic acid solution from which the high-boiling point by-products were separated was mixed with the absorption tower side discharge stream to obtain a mixed stream. The mixed stream included acrylic acid (88.8 wt%), acetic acid (2.98 wt%), and water (5.7 wt%).

Specifically, the mixed stream was supplied to the crystallizer (a) of the first stage crystallization unit and the crystallization process was performed. Thus, a suspension including the acrylic acid crystal was obtained. At this time, the crystallization temperature in the crystallizer (a) of the first stage crystallization unit was -2°C. A ratio of the mass of the acrylic acid crystal included in the suspension to the mass of the suspension discharged from the crystallizer (a) of the first stage crystallization unit was 30 wt%.

Thereafter, the suspension obtained from the crystallizer (a) of the first stage crystallization unit was supplied to the solid-liquid separator (b) of the first stage crystallization unit. In the solid-liquid separator (b) of the first stage crystallization unit, the suspension obtained from the crystallizer (a) of the first stage crystallization unit was separated into an acrylic acid crystal and a mother liquor. At this time, in the solid-liquid separator (b) of the first stage crystallization unit, the solid-liquid separation of the suspension was performed by filtration.

The mother liquor separated from the solid-liquid separator (b) of the first stage crystallization unit diverged into a first mother liquor stream and a second mother liquor stream, which were circulated to the absorption tower 100 and the crystallizer (a) of the first stage crystallization unit, respectively. At this time, the first mother liquor stream diverging from the first stage solid-liquid separator was circulated to 26% height from the top to the bottom of the absorption tower, based on the top of the absorption tower 100 being 0%. In addition, the mass flow rate of the first mother liquor stream diverging from the first stage solid-liquid separator was 16% of the mass flow rate of the mixed gas. The mass flow rate of the first mother liquor stream diverging from the first stage solid-liquid separator was 99.2% of the mass flow rate of the acrylic acid crystal separated from the solid-liquid separator (b) of the second stage crystallization unit described later.

Meanwhile, the mass flow rate ratio between the first mother liquor stream and the second mother liquor stream diverging from the first stage solid-liquid separator was 1.7:8.3. The first mother liquor stream diverging from the first stage solid-liquid separator included acrylic acid (78.2 wt%), acetic acid (5.5 wt%), and water (11.2 wt%).

Meanwhile, the acrylic acid crystal separated from the solid-liquid separator (b) of the first stage crystallization unit was melted to obtain a concentrated acrylic acid solution. The concentrated acrylic acid solution originating from the solid-liquid separator (b) of the first stage crystallization unit was supplied to the crystallizer (a) of the second stage crystallization unit.

The suspension including the acrylic acid crystal was obtained from the crystallization process performed in the crystallizer (a) of the second stage crystallization unit. At this time, the crystallization temperature in the crystallizer (a) of the second stage crystallization unit was 9.5°C. A ratio of the mass of the acrylic acid crystal included in the suspension to the mass of the suspension discharged from the crystallizer (a) of the second stage crystallization unit was 30 wt%.

Thereafter, the suspension obtained from the crystallizer (a) of the second stage crystallization unit was supplied to the solid-liquid separator (b) of the second stage crystallization unit. In the solid-liquid separator (b) of the second stage crystallization unit, the suspension obtained from the crystallizer (a) of the second stage crystallization unit was separated into an acrylic acid crystal and a mother liquor. At this time, in the solid-liquid separator (b) of the second stage crystallization unit, the solid-liquid separation of the suspension was performed by filtration.

The mother liquor separated from the solid-liquid separator (b) of the second stage crystallization unit diverged into the first mother liquor stream and the second mother liquor stream, which were circulated to the crystallizer of the first stage crystallization unit and the crystallizer of the second stage crystallization unit, respectively. At this time, the mass flow rate ratio between the first mother liquor stream and the second mother liquor stream diverging from the solid-liquid separator (b) of the second stage crystallization unit was 6.7:3.3.

Meanwhile, the acrylic acid crystal separated from the solid-liquid separator (b) of the second stage crystallization unit was melted to obtain a concentrated acrylic acid solution, and a purified acrylic acid was obtained therefrom.

As a result, the purified acrylic acid included acrylic acid (99.4 wt%), acetic acid (0.4 wt%), and water (0.1 wt%). In addition, the amount of the acrylic acid included in the absorption tower upper discharge stream (acrylic acid loss) was 0.137 mol%. At this time, the mol% of the acrylic acid is a value which is a ratio of the moles of acrylic acid to the total moles of the absorption tower upper discharge stream (gas phase), expressed as %.

### Example 2

In Example 2, the crystallization temperature in the crystallizer of the first stage crystallization unit was - 5°C, and the crystallization temperature of the second stage crystallization unit was 8.5°C, as compared with Example 1.

In addition, the first mother liquor stream diverging from the first stage solid-liquid separator included acrylic acid (73.7 wt%), acetic acid (6.5 wt%), and water (13.7 wt%). The mass flow rate of the first mother liquor stream diverging from the first stage solid-liquid separator was 11% of the mass flow rate of the mixed gas. The mass flow rate ratio between the first mother liquor stream and the second mother liquor stream diverging from the first stage solid-liquid separator was 1.4:8.6. The mass flow rate of the first mother liquor stream diverging from the first stage solid-liquid separator was 70.3% of the mass flow rate of the acrylic acid crystal separated from the solid-liquid separator (b) of the second stage crystallization unit.

The mass flow rate ratio between the first mother liquor stream and the second mother liquor stream diverging from the solid-liquid separator (b) of the second stage crystallization unit was 5.5:4.5.

As a result, the concentrated acrylic acid solution obtained by melting the acrylic acid crystal obtained from the solid-liquid separator of the second stage crystallization unit, that is, the purified acrylic acid included acrylic acid (99.2 wt%), acetic acid (0.52 wt%), and water (0.18 wt%). In addition, the amount of the acrylic acid included in the absorption tower upper discharge stream (acrylic acid loss) was 0.133 mol%.

### Example 3

In Example 3, the crystallization temperature in the crystallizer of the first stage crystallization unit was - 8°C, and the crystallization temperature of the second stage crystallization unit was 8°C, as compared with Example 1.

In addition, the first mother liquor stream diverging from the first stage solid-liquid separator included acrylic acid (69.6 wt%), acetic acid (7.3 wt%), and water (16.0 wt%). The mass flow rate of the first mother liquor stream diverging from the first stage solid-liquid separator was 9% of the mass flow rate of the mixed gas. The mass flow rate ratio between the first mother liquor stream and the second mother liquor stream diverging from the first stage solid-liquid separator was 1.1:8.9. The mass flow rate of the first mother liquor stream diverging from the first stage solid-liquid separator was 54.9% of the mass flow rate of the acrylic acid crystal separated from the solid-liquid separator (b) of the second stage crystallization unit described later.

The mass flow rate ratio between the first mother liquor stream and the second mother liquor stream diverging from the solid-liquid separator (b) of the second stage crystallization unit was 5.7:4.3.

As a result, the concentrated acrylic acid solution obtained by melting the acrylic acid crystal obtained from the solid-liquid separator of the second stage crystallization unit, that is, the purified acrylic acid included acrylic acid (99.2 wt%), acetic acid (0.57 wt%), and water (0.2 wt%). In addition, the amount of the acrylic acid included in the absorption tower upper discharge stream (acrylic acid loss) was 0.131 mol%.

### Comparative Examples

### Comparative Example 1

According to the process diagram shown in FIG. 2, a preparation process of an acrylic acid was simulated, using an Aspen Plus simulator available from Aspen Technology, Inc.

In Comparative Example 1, the mother liquors separated from the solid-liquid separators (b) of the first stage crystallization unit and the second stage crystallization unit, respectively did not diverge and were supplied to the absorption tower 100 and the crystallizer (a) of the first stage crystallization unit, respectively.

Specifically, a mixed gas having the same composition as in Example 1 was supplied to the absorption tower 100 and was brought into contact with water as an absorption solvent to obtain an acrylic acid solution. The acrylic acid solution included acrylic acid (89.3 wt%), acetic acid (3.0 wt%), and water (5.4 wt%).

The acrylic acid solution was introduced to the crystallizer (a) of the first stage crystallization unit to obtain a suspension including an acrylic acid crystal. At this time, the crystallization temperature in the crystallizer (a) of the first stage crystallization unit was 3.6°C. The suspension obtained from the crystallizer (a) of the first stage crystallization unit was supplied to the solid-liquid separator (b) of the first stage crystallization unit. At this time, a ratio of the mass of the acrylic acid crystal included in the suspension to the mass of the suspension discharged from the crystallizer (a) of the first stage crystallization unit was 30 wt%.

In the solid-liquid separator (b) of the first stage crystallization unit, the suspension obtained from the crystallizer (a) of the first stage crystallization unit was separated into an acrylic acid crystal and a mother liquor. At this time, in the solid-liquid separator (b) of the first stage crystallization unit, the solid-liquid separation of the suspension was performed by filtration.

Meanwhile, the acrylic acid crystal separated from the solid-liquid separator (b) of the first stage crystallization unit was melted to obtain a concentrated acrylic acid solution. The concentrated acrylic acid solution originating from the solid-liquid separator (b) of the first stage crystallization unit was supplied to the crystallizer (a) of the second stage crystallization unit, and the mother liquor separated from the solid-liquid separator (b) of the first stage crystallization unit was supplied to the absorption tower 100. At this time, the mother liquor separated from the solid-liquid separator (b) of the first stage crystallization unit included acrylic acid (87.9 wt%), acetic acid (3.4 wt%), and water (6.2 wt%). The mass flow rate of the mother liquor separated from the solid-liquid separator (b) of the first stage crystallization unit was 762.1% of the mass flow rate of the acrylic acid crystal separated from the solid-liquid separator (b) of the second stage crystallization unit.

In the crystallizer (a) of the second stage crystallization unit, the concentrated acrylic acid solution originating from the solid-liquid separator (b) of the first stage crystallization unit was crystallized to obtain a suspension including the acrylic acid crystal. At this time, the crystallization temperature in the crystallizer (a) of the second stage crystallization unit was 11.7°C. A ratio of the mass of the acrylic acid crystal included in the suspension to the mass of the suspension discharged from the crystallizer (a) of the second stage crystallization unit was 30 wt%.

The suspension obtained from the crystallizer (a) of the second stage crystallization unit was supplied to the solid-liquid separator (b) of the second stage crystallization unit. In the solid-liquid separator (b) of the second stage crystallization unit, the suspension obtained from the crystallizer (a) of the second stage crystallization unit was separated into an acrylic acid crystal and a mother liquor. At this time, in the solid-liquid separator (b) of the second stage crystallization unit, the solid-liquid separation of the suspension was performed by filtration.

Meanwhile, the mother liquor separated from the solid-liquid separator (b) of the second stage crystallization unit was supplied to the crystallizer (a) of the first stage crystallization unit The acrylic acid crystal separated from the solid-liquid separator (b) of the second stage crystallization unit was melted to obtain a concentrated acrylic acid solution, and a purified acrylic acid was obtained therefrom.

As a result, the purified acrylic acid included acrylic acid (99.6 wt%), acetic acid (0.23 wt%), and water (0.07 wt%). In addition, the amount of the acrylic acid included in the absorption tower upper discharge stream (acrylic acid loss) was 0.175 mol%.

In Comparative Example 1, though the flow rate of the mother liquor separated from the solid-liquid separator (b) of the first stage crystallization unit was higher than the flow rate of the acrylic acid crystal separated from the solid-liquid separator (b) of the second stage crystallization unit, the amount of the acrylic acid included in the mother liquor separated from the solid-liquid separator (b) of the first stage crystallization unit was also high, and thus, it was confirmed that the amount of the acrylic acid lost through the absorption tower upper discharge stream was the highest. In addition, since the flow rate of the mother liquor separated from the solid-liquid separator (b) of the first stage crystallization unit was high, a problem in which the device size, for example, the sizes of the crystallizer of the first stage crystallization unit and the solid-liquid separator should be increased may arise.

### Comparative Example 2

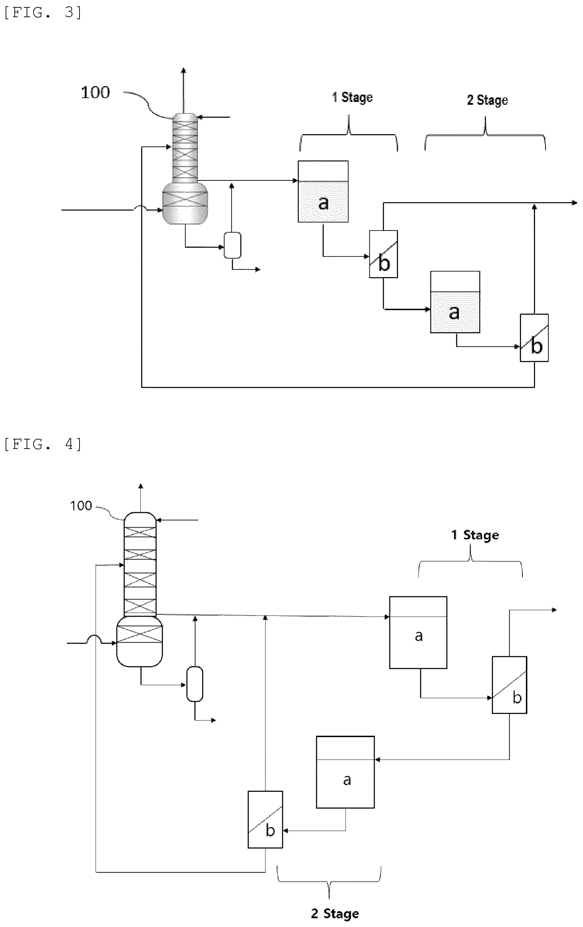

According to the process diagram shown in FIG. 3, a preparation process of an acrylic acid was simulated, using an Aspen Plus simulator available from Aspen Technology, Inc.

Specifically, a mixed gas having the same composition as in Example 1 was supplied to the absorption tower 100 and was brought into contact with water as an absorption solvent to obtain an acrylic acid solution. The acrylic acid solution included acrylic acid (88.8 wt%), acetic acid (2.98 wt%), and water (6.2 wt%).

The acrylic acid solution was crystallized in the crystallizer (a) of the first stage crystallization unit to obtain a suspension including an acrylic acid crystal. At this time, the crystallization temperature in the crystallizer (a) of the first stage crystallization unit was 1.6°C. A ratio of the mass of the acrylic acid crystal included in the suspension to the mass of the suspension discharged from the crystallizer (a) of the first stage crystallization unit was 30 wt%.

The suspension obtained from the crystallizer (a) of the first stage crystallization unit was supplied to the solid-liquid separator (b) of the first stage crystallization unit. In the solid-liquid separator (b) of the first stage crystallization unit, the suspension obtained from the crystallizer (a) of the first stage crystallization unit was separated into an acrylic acid crystal and a mother liquor. Meanwhile, the acrylic acid crystal separated from the solid-liquid separator (b) of the first stage crystallization unit was melted to obtain a concentrated acrylic acid solution.

The mother liquor separated from the solid-liquid separator (b) of the first stage crystallization unit was supplied to the crystallizer (a) of the second stage crystallization unit and crystallized, thereby obtaining a suspension including an acrylic acid crystal. At this time, the crystallization temperature in the crystallizer (a) of the second stage crystallization unit was -1.7°C. A ratio of the mass of the acrylic acid crystal included in the suspension to the mass of the suspension discharged from the crystallizer (a) of the second stage crystallization unit was 30 wt%.

The suspension obtained from the crystallizer (a) of the second stage crystallization unit was supplied to the solid-liquid separator (b) of the second stage crystallization unit, and separated into an acrylic acid crystal and a mother liquor. The acrylic acid crystal separated from the solid-liquid separator (b) of the second stage crystallization unit was melted to obtain a concentrated acrylic acid solution.

The mother liquor separated from the solid-liquid separator (b) of the second stage crystallization unit was circulated to the absorption tower 100. The mother liquor separated from the solid-liquid separator (b) of the second stage crystallization unit included acrylic acid (79.0 wt%), acetic acid (5.1 wt%), and water (12.0 wt%). The mass flow rate of the mother liquor separated from the solid-liquid separator (b) of the second stage crystallization unit was 92.4% of the mass flow rate of the acrylic acid crystal separated from the solid-liquid separator (b) of the second stage crystallization unit.

Meanwhile, the concentrated acrylic acid solution separated from the solid-liquid separator (b) of the second stage crystallization unit formed a mixed stream with the concentrated acrylic acid solution separated from the solid-liquid separator (b) of the first stage crystallization unit, and the mixed stream was obtained as a purified acrylic acid.

As a result, the purified acrylic acid included acrylic acid (97.9 wt%), acetic acid (1.0 wt%), and water (0.86 wt%). In addition, the amount of the acrylic acid included in the absorption tower upper discharge stream (acrylic acid loss) was 0.135 mol%.

In Comparative Example 2, though the amount of the acrylic acid lost through the absorption tower upper discharge stream was similar to that of the examples, it became poor as compared with the examples, in terms of the purity of the purified acrylic acid, that is, the content of the acrylic acid included in the purified acrylic acid.

Meanwhile, in Examples 1 to 3 of the present invention, the concentrated acrylic acid solution was obtained in the crystallization unit, and the concentrated acrylic acid solution was supplied to the crystallization unit in the after-stage and additional crystallization was performed, but the mother liquor separated from the solid-liquid separator of each stage was not supplied to the after-stage. That is, Examples 1 to 3 suggest an embodiment in which acrylic acid crystal content in the acrylic acid solution was increased toward the after-stage, and finally, the purified acrylic acid was obtained from the acrylic acid solution discharged from the crystallization unit in the last stage.

However, the following Comparative Examples 3 to 5 were similar to Examples 1 to 3 in that the crystallization units including the crystallizer and the solid-liquid separator were included in multiple stages, but are an embodiment regarding a method in which the solid-liquid separated acrylic acid solution was supplied to the crystallizer in the corresponding stage or the crystallizer in the prior-stage to obtain a purified acrylic acid in the first stage crystallization unit, but since the solid-liquid separated mother liquor was supplied to the crystallization unit in the after-stage to go through stages, thereby concentrating the mother liquor.

### Comparative Example 3

In Comparative Example 3, according to the process diagram shown in FIG. 4, as in Example 1, a preparation process of an acrylic acid was simulated, using an Aspen Plus simulator available from Aspen Technology, Inc.

As in Example 1, the side discharge stream from the absorption tower and the upper discharge stream from the high-boiling point by-product removal tower which went through the same process as in Example 1 were mixed, and a mixed stream including acrylic acid (88.8 wt%), acetic acid (2.98 wt%), and water (5.7 wt%) was introduced to the multistage crystallization process in which a total of two stages of crystallization units were connected.

As shown in FIG. 4, in Comparative Example 3, the mother liquor separated from the solid-liquid separator of the first stage crystallization unit was supplied to the crystallizer (a) of the second crystallization unit to perform additional crystallization, and the acrylic acid solution separated from the solid-liquid separator (b) of the second crystallization unit was circulated to the crystallizer (a) of the first stage crystallization unit. The acrylic acid crystal separated from the solid-liquid separator (b) of the first stage crystallization unit was melted to obtain a concentrated acrylic acid solution, and a purified acrylic acid was obtained therefrom.

Specifically, crystallization was performed at a crystallization temperature in the crystallizer (a) of the first stage crystallization unit of -2°C as in Example 1, and as a result, a ratio of the mass of the acrylic acid crystal included in the suspension to the mass of the suspension discharged from the crystallizer (a) of the first stage crystallization unit was 55 wt%, which was in a very high level, and as a result, it was difficult to mix the suspension in the crystallizer (a) of the first stage crystallization unit and the purified acrylic acid was not able to be obtained by a subsequent process.

### Comparative Example 4

In Comparative Example 4, as in Comparative Example 3, according to the process diagram shown in FIG. 4, the crystallization temperature of the crystallizer (a) of the first stage crystallization unit was controlled to 3.3°C so that a ratio of the mass of the acrylic acid crystal included in the suspension to the mass of the suspension discharged from the crystallizer (a) of the first stage crystallization unit was 30 wt%.

The suspension discharged from the crystallizer (a) of the first stage crystallization unit was solid-liquid separated from the solid-liquid separator (b) of the first stage crystallization unit, but the mother liquor was supplied to the crystallizer (a) of the second stage crystallization unit, and the purified acrylic acid was obtained from the concentrated acrylic acid solution.

Additional crystallization for the mother liquor was performed in the crystallizer (a) of the second stage crystallization unit, and the mass ratio of the acrylic acid crystal in the discharged suspension was 30 wt%. In order to maintain the mass ratio of the acrylic acid crystal in the discharge suspension at 30 wt% as in Example 1, the crystallization temperature of the crystallizer (a) of the second stage crystallization unit was controlled to 0.7°C.

The suspension discharged from the crystallizer (a) of the second stage crystallization unit was solid-liquid separated in the solid-liquid separator (b) of the second stage crystallization unit. The concentrated acrylic acid solution separated from the solid-liquid separator (b) of the second crystallization unit was circulated to the crystallizer (a) of the first crystallization unit, and the separated mother liquor was supplied to the absorption tower 100.

Meanwhile, the acrylic acid crystal separated from the solid-liquid separator (b) of the first stage crystallization unit was melted to obtain a concentrated acrylic acid solution, and a purified acrylic acid was obtained therefrom. As a result, the purified acrylic acid included acrylic acid (98.5 wt%), acetic acid (0.75 wt%), and water (0.6 wt%).

The amount of the acrylic acid included in the absorption tower upper discharge stream (acrylic acid loss) was 0.144 mol%.

As a result, in Comparative Example 4, it was confirmed that the purity of the purified acrylic acid (content of acrylic acid) was lowered as compared with Example 1, and the loss amount of the acrylic acid lost through the absorption tower upper portion discharge stream was increased.

### Comparative Example 5

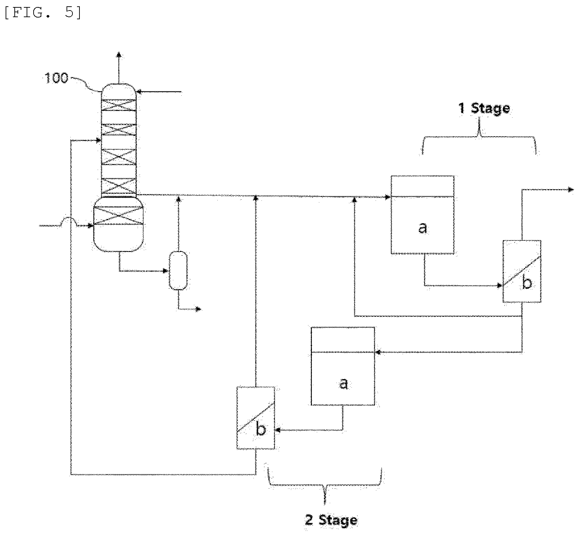

In Comparative Example 5, according to the process diagram shown in FIG. 5, as in Example 1, a preparation process of an acrylic acid was simulated, using an Aspen Plus simulator available from Aspen Technology, Inc.

In Comparative Example 5, an acrylic acid was prepared in the same manner as in Comparative Example 4, except that a part of the mother liquor of the solid-liquid separator (b) of the first stage crystallization unit diverged and supplied to the crystallizer of the first stage crystallization unit, and the rest of the mother liquor was supplied to the crystallizer of the second stage crystallization unit.

In order to set the temperature of the crystallizer (a) of the first stage crystallization unit to -2°C as in Example 1 and maintain the mass ratio of the acrylic acid crystal in the suspension discharged from each crystallizers (a) in the first and second stages at 30 wt% as in Example 1, the flow rate of the mother liquor supplied to the crystallizer (a) of the first stage crystallization unit of the mother liquor of the solid-liquid separator (b) of the first stage crystallization unit was 2.3 times the flow rate of the mother liquor supplied to the crystallizer (a) of the second stage crystallization unit. Meanwhile, the acrylic acid crystal separated from the solid-liquid separator (b) of the first stage crystallization unit was melted to obtain a concentrated acrylic acid solution, and a purified acrylic acid was obtained therefrom. As a result, the purified acrylic acid included acrylic acid (97.5 wt%), acetic acid (1.14 wt%), and water (1.19 wt%).

The amount of the acrylic acid included in the absorption tower upper discharge stream (acrylic acid loss) was 0.132 mol%.

As a result, in Comparative Example 5, it was found that the amount of the acrylic acid lost as the absorption tower upper portion discharge stream was equivalent to Example 1, but the content (purity) of the acrylic acid in the purified acrylic acid obtained from the solid-liquid separator (b) of the first stage crystallization unit was lowered.

### [Description of symbols]

(a): crystallizer
(b): solid-liquid separator
100: absorption tower

## Claims

1. A method for preparing an acrylic acid, the method comprising:
bringing a mixed gas including an acrylic acid into contact with water in an absorption tower to obtain an acrylic acid solution including the acrylic acid;
performing crystallization of the acrylic acid by a process including a multistage crystallization process in which two or more crystallization units including a crystallizer and a solid-liquid separator are connected in series,
introducing the acrylic acid solution to the crystallizer of a first stage crystallization unit of the two or more crystallization units;
crystallizing the acrylic acid, which included in the acrylic acid solution introduced to the crystallizer in each stage, in the crystallizer in each stage to obtain a suspension including an acrylic acid crystal, and supplying the suspension to a solid-liquid separator in each stage;
solid-liquid separating the suspension in the solid-liquid separator in each stage to obtain a concentrated acrylic acid solution and a mother liquor;
diverging the mother liquor into a first mother liquor stream and a second mother liquor stream, circulating the first mother liquor stream to the crystallizer in a prior-stage to a stage to which the solid-liquid separator from which the mother liquor is separated belongs or to the absorption tower, and circulating the second mother liquor stream to the crystallizer in the stage to which the solid-liquid separator from which the mother liquor is separated belongs;
introducing the concentrated acrylic acid solution to the crystallizer in an after-stage of a stage to which the solid-liquid separator from which the concentrated acrylic acid solution is separated belongs; and
obtaining a purified acrylic acid from the solid-liquid separator of the crystallization unit in the last stage of the two or more crystallization units.

2. The method for preparing an acrylic acid of claim 1, wherein a amount of the acrylic acid included in the acrylic acid solution introduced to a crystallizer of the first stage crystallization unit is 80 to 95 wt%.

3. The method for preparing an acrylic acid of claim 1, wherein a ratio of a mass of the acrylic acid crystal included in the suspension to a mass of the suspension discharged from the crystallizer in each stage is 10 to 40 wt%.

4. The method for preparing an acrylic acid of claim 1, wherein the mother liquor obtained from the solid-liquid separator of the first stage crystallization unit diverges into the first mother liquor stream and the second mother liquor stream, the first mother liquor stream diverging from the first stage solid-liquid separator is circulated to the absorption tower, and the second mother liquor stream diverging from the first stage solid-liquid separator is circulated to the crystallizer of the first stage crystallization unit.

5. The method for preparing an acrylic acid of claim 4, wherein a position at which the first mother liquor stream diverging from the first stage solid-liquid separator is circulated to the absorption tower is at 10% to 60% height from the top to the bottom of the absorption tower, based on the top of the absorption tower being 0%.

6. The method for preparing an acrylic acid of claim 4, wherein a mass flow rate of the first mother liquor stream diverging from the first stage solid-liquid separator is 5 to 50% of a mass flow rate of the mixed gas including the acrylic acid.

7. The method for preparing an acrylic acid of claim 1,
wherein the two or more crystallization units are in a total of two stages,
a crystallization temperature in the crystallizer of the first stage crystallization unit is -9 to 5°C, and
a crystallization temperature in the crystallizer of the second stage crystallization unit is 0 to 12°C.

8. The method for preparing an acrylic acid of claim 7,
wherein a mass flow rate between the first mother liquor stream and the second mother liquor stream diverging from the solid-liquid separator of the first stage crystallization unit is 1:9 to 1:1, and
a mass flow rate between the first mother liquor stream and the second mother liquor stream diverging from the solid-liquid separator of the second stage crystallization unit is 1:1 to 9:1.

9. The method for preparing an acrylic acid of claim 1, wherein the mixed gas including the acrylic acid is a reaction product by a gaseous oxidation reaction of reactants including gas containing oxygen and a raw material compound.

10. The method for preparing an acrylic acid of claim 9, wherein the raw material compound is one or more compounds selected from the group consisting of propane, propylene, butane, i-butylene, t-butylene, and acrolein.

11. The method for preparing an acrylic acid of claim 1,
wherein the obtaining of a concentrated acrylic acid solution in the solid-liquid separator in each stage is performed by including:
solid-liquid separating the suspension in the solid-liquid separator in each stage to obtain the acrylic acid crystal and melting the acrylic acid crystal to obtain the concentrated acrylic acid solution.
